Europäisches Patentamt

European Patent Office

Office européen des brevets

(19)

(11) Veröffentlichungsnummer : **0 427 360 B1**

(12) **EUROPÄISCHE PATENTSCHRIFT**

(45) Veröffentlichungstag der Patentschrift :
15.06.94 Patentblatt 94/24

(21) Anmeldenummer : 90250271.5

(22) Anmeldetag : 23.10.90

(51) Int. Cl.⁵ : **A61K 49/02**

(54) **Kit (nichtradioaktive Vorstufe) zur Herstellung einer enantiomeren Form des Nierenfunktionsdiagnostikums Technetium-99m-Mercaptoacetyltriglycin (99m-Tc-MAG-3) und Verfahren zur Herstellung des Kits.**

(30) Priorität : 30.10.89 DD 334034
30.10.89 DD 334035

(43) Veröffentlichungstag der Anmeldung :
15.05.91 Patentblatt 91/20

(45) Bekanntmachung des Hinweises auf die
Patenterteilung :
15.06.94 Patentblatt 94/24

(84) Benannte Vertragsstaaten :
AT DE FR GR IT NL

(56) Entgegenhaltungen :
EP-A- 0 135 160
EP-A- 0 200 492
EP-A- 0 284 071
US-A- 4 670 545
J. NUCL. MED., Band 27, Nr. 1, Januar 1986,
Seiten 111-116, New York, US; A.R. FRITZ-
BERG et al.

(73) Patentinhaber : **Verein für
Kernverfahrenstechnik und Analytik
Rossendorf e.V.
Postfach 51 01 19
D-01314 Dresden (DE)**

(72) Erfinder : **Noll, Bernhard, Dr. rer. nat.
Dresdner Strasse 104
O-8210 Freital (DE)**
Erfinder : **Johannsen, Bernd, Dr. sc.
Wilhelm-Pieck-Strasse 72
O-1054 Berlin (DE)**
Erfinder : **Münze, Rudolph, Prof. Dr.
Wetrower Strasse
O-8051 Dresden (DE)**
Erfinder : **Spies, Hartmut, Dr.sc.
Räcknitzhöhe 49
O-8020 Dresden (DE)**

## Beschreibung

Die Erfindung betrifft einen Kit (eine nichtradioaktive Vorstufe) zur Herstellung einer stabilen enantiomeren Form des Nierenfunktionsdiagnostikums Technetium-99m-Mercaptoacetyltriglycin ([99mTc-MAG-3) für nuklearmedizinische Untersuchungen der Nierenfunktion, sowie ein Verfahren zur Herstellung dieses Kits.

Voraussetzung für eine breite klinische Nutzung des Nierenfunktionsdiagnostikums Technetium-99m-Mercaptoacetyltriglycin ( [99mTc-MAG-3 ) auf Basis eines Kits ist ein Verfahren, das die Bildung einer stabilen Komplexverbindung des Mercaptoacetyltriglycins mit dem Technetium auf der Oxidationsstufe +5 ermöglicht. Die Markierung soll nach Möglichkeit ohne Einschränkung hinsichtlich der spez. Aktivität, des Alters und Volumens des Generatoreluats bei Raumtemperatur in einem Schritt durchführbar sein. Das derartige Präparat muß ohne anschließende präparative Trennoperation in der klinischen Routineanwendung einsetzbar sein. Diese Bedingungen werden von den aus der Literatur bekannten Verfahren nur zum Teil erfüllt .

Bei dem von Fritzberg [vgl. US-PS 4 670 545 ] beschriebenen Verfahren unter Verwendung von 1,2-bis-(Mercaptoacetamido)ethan- Derivaten muß aufgrund der Bildung von Isomeren, die in vivo ein unterschiedliches Verhalten zeigen, eine Trennoperation mittels HPLC angeschlossen werden.

Bei der Herstellung des nierenaffinen [99mTc-Mercaptoacetyltriglycins ([99mTc-MAG-3) unter Verwendung des S-benzoyl-geschützten Liganden Benzoyl-MAG-3 [vgl. EP-A-0173 424 / Produkt "TechneScan MAG3" der Fa. Mallinckrodt ] werden Einschränkungen hinsichtlich der Eluatmenge und der Art der Zubereitung derart angegeben, daß nur frisches Eluat von maximal 1ml in einer 1:3 Verdünnung eingesetzt werden kann. Das Reaktionsgemisch muß 10 min. im kochenden Wasserbad erhitzt und anschließend durch Kühlung wieder auf Zimmertemperatur gebracht werden. Aufgrund einer durch die Erhitzung bewirkten Weiterführung der Reduktion des Tc(V)-MAG-3 zum Tc(IV)-MAG-3 im pH-Bereich dieser Zubereitung infolge überschüssigen Zinn(II)-chlorids wird nach neuesten Berichten [vgl. D.L.Nosco et al./ Mallinckrodt Medical/ "Third International Symposium On Technetium In Chemistry And Nuclear Medicine", Padua 1989 ] das Durchblasen von mindestens 2ml steriler Luft gefordert, um den Tc(IV)MAG-3 Komplex wieder zum Tc(V)MAG-3-Komplex zu oxidieren. Die Handhabung dieses Verfahrens ist damit für den klinischen Routinebetrieb sowohl vom Strahlenschutz als auch der chemischen Reaktionsführung her relativ aufwendig, und weist zahlreiche potentielle Fehlerquellen auf.

Darüberhinaus wurde durch von Verbrüggen [vgl. Nuclear Medicine,Trends and Possibilities in Nuclear Medicine,Stuttgart-New York , 1989,S.436-439 ] die Existenz von zwei enantiomeren Former des [99mTc-MAG-3 in dem nach dem genannten Verfahren hergestellten Produkt nachgewiesen. Diese beiden stereoisomeren Formen lassen sich nicht auf chemischem Wege trennen, können aber möglicherweise unterschiedliches Bioverhalten zeigen.

In der Gebrauchsinformation zu dem auf dem o.g. Verfahren basierenden Kit "TechneScan MAG3" (DRN 4334) wird für das frisch hergestellte Produkt eine Markierungsausbeute $\geq$ 95% angegeben und diese nur für eine Stund garantiert. Die im frischen Markierungsansatz enthaltene und mit der Zeit zunehmende Verunreinigung wird in der Leber gespeichert und über die Gallenblase ausgeschieden, und kann so die späte Phase (nach 30 min) einer dynamischen Nierenuntersuchung beeinflussen.

Der Erfindung lag daher die Aufgabe zugrunde, einen Kit zur Herstellung des Nierenfunktionsdiagnostikums [99mTc-MAG-3 so zu gestalten, daß man eine stabile enantiomere Form der [99mTc-Mercaptoacetyltriglycinverbindung mit hoher Organaffinität und dem erforderlichen Bioverhalten durch einfache Zugabe von Technetium-99m-Generatoreluat zu einer gebrauchsfertigen Zubereitung aus Reduktionsmittel und Ligand bei Zimmertemperatur ohne nachfolgenden Reinigungsschritt erhält. Diese Aufgabe schloß ein Verfahren zur Herstellung eines solchen Kits mit ein.

Erfindungsgemäß wurde die Aufgabe mit einem Kit gelöst, der aus zwei Komponenten besteht, deren erste ein lyophylisiertes Gemisch aus Mercaptoacetyltriglycin, einen die Oxidationsstufe +5 des Tc stabilisierenden Koliganden, ein Reduktionsmittel und ein Alkali- bzw. Erdalkalihydroxid im Molverhältnis

```
    1    :  ( 50 - 150 )  :    ( 0,1 - 0,5)    :  ( 30 - 80 )
  MAG-3  :    Koligand     :   Reduktionsmittel :     Hydroxid
```

und deren zweite Komponente eine sterile Phosphatpufferlösung mit einer dem Molverhältnis MAG-3 : Hydroxid entsprechenden Menge Säure enthält.

Dabei wird in bevorzugter Ausgestaltung als Koligand ein Salz der Weinsäure oder der Gluconsäure, insbesondere $Na_2$-Tartrat, als Reduktionsmittel $SnCl_2$ und als Hydroxid NaOH eingesetzt.

Eine vorteilhafte, auf die in den nuklearmedizinischen Einrichtungen vorwiegend eingesetzten [99Mo / [99mTc - Generatoren abgestimmte Ausführungsform des erfindungsgemäßen Kits besteht darin, daß die erste Komponente aus einem lyophylisierten Gemisch aus

0,2 mg MAG-3

22 mg Dinatriumtartrat x $2H_2O$

60 µg $SnCl_2$ x $2H_2O$

1,72 mg NaOH besteht.

Das Verfahren zur Herstellung des Kits besteht darin, daß zur Herstellung der ersten Komponente Benzoyl-MAG-3 mit einem Alkoholat in alkoholischer Lösung verseift und der so gewonnene Ligand Mercaptoacetyltriglycin mit in ungeschützter Form vorliegender Mercaptogruppe mit dem Reduktionsmittel und dem die Oxidationsstufe +5 des Tc stabilisierenden Koliganden bei einem pH-Wert > 11 im oben angegeben Verhältnis gemischt, lyophylisiert und in Portionen abgefüllt wird.

Vorteilhafterweise erfolgt dabei die Verseifung des Benzoyl-MAG-3 mit $NaOCH_3$ in methanolischer Lösung unter einem Schutzgas.

In der klinischen Praxis wird dieser Kit derart eingesetzt, daß die Komponente 1 mit $^{99m}$Tc-Eluat versetzt, und nach einer Reaktionszeit von ca. 15 Minuten die Komponente 2 (Pufferlösung) zugesetzt wird. Die so entstehende Lösung ist sofort injektionsfertig.

Es ist hierbei nicht erforderlich, das Reaktionsgemisch zu erhitzen, was für den klinischen Routinebetrieb sowohl vom Strahlenschutz als auch von der chemischen Reaktionsführung einen wesentlichen Vorteil bildet. Durch die Verwendung des S-ungeschützten Mercaptoacetyltriglycins und die Einstellung eines pH- Wertes > 11 werden Bedingungen geschaffen, die zur bevorzugten Bildung einer stabilen enantiomeren Form des Technetium-99m-MAG-3-Komplexes führen.

Es ist keine Einschränkung hinsichtlich der einsetzbaren Aktivitätsmenge erforderlich. Das hat zur Folge, daß die Patientenzahl, die mit einem Ansatz untersucht werden kann, keiner engen Begrenzung unterliegt.

Für das der Erfindung zugrundeliegende Verfahren gibt es auch keine Einschränkung hinsichtlich des möglichen Gesamtvolumens an $^{99m}$Tc-Eluat.

Die Reinheit des nach der Erfindung hergestellten Präparates beträgt >98% und ist in dem gesamten, nur durch das Abklingverhalten des $^{99m}$Tc begrenzten Zeitraum stabil.

Nachfolgend soll die Erfindung anhand eines Ausführungsbeispieles näher erläutert werden.

Der erfindungsgemäße Kit besteht z.B. für ein in der Klinik einzusetzendes Volumen an $^{99m}$Tc-Generatoreluat von 1-4 ml aus folgenden zwei Abpackungen :

Komponente 1 :  lyophylisiertes Gemisch aus
0,2 mg MAG-3
22 mg Dinatriumtartrat x $2H_2O$
60 µg $SnCl_2$ x $2H_2O$
1,72 mg NaOH

Komponente 2 :  sterile Pufferlösung aus
1,638 ml 0,1 m $Na_2HPO_4$
0,382 ml 0,1 m $NaH_2PO_4$
0,04 ml 1 n HCl

Die Herstellung dieser konkreten Ausführungsform des erfindungsgemäßen Kits kann für einen Produktionsumfang von je 150 Flaschen der Komponenten 1 und 2 z.B. folgendermaßen geschehen:

Für die erste Komponente werden die folgenden Stammlösungen eingesetzt:

Stammlösung A :  30 mg MAG-3 in 6 ml dest.Wasser

Stammlösung B :  3,30 g Natriumtartrat in 18 ml dest.Wasser

Stammlösung C :  4,50 ml $SnCl_2$-Lsg. (200 mg $SnCl_2$/100 ml 0,1n HCl)

Stammlösung D :  32,3 ml 0,2 n NaOH

Diese Stammlösungen werden vereinigt und anschließend durch ein Sterilfilter in ein Vorratsgefäß filtriert und dort zugekühlt. Davon werden 400 µl pro Injektionsflasche dosiert. Danach werden die LÖsungen lyophilisiert und die Flaschen verschlossen.

Für die zweite Komponente (Pufferlösung) werden 81,9 ml 0,1 m $Na_2HPO_4$-Lösung und 19,1 ml 0,1m $NaH_2PO_4$-Lösung gemischt, mit 2ml 1n HCl versetzt und sterilisiert. Anschließend werden davon pro Flasche 2ml abgefüllt.

Bei der klinischen Anwendung werden zwecks radioaktiver Markierung zur lyophilisierten Komponente 1 des Kits je nach Bedarf 1-4 ml $^{99m}$Tc-Generatoreluat mit Hilfe einer Injektionsspritze zugegeben . Nach einer Reaktionszeit von 15 min. werden die gesamten 2ml der sterilen isotonischen Phosphatpufferlösung zugesetzt. Die so erhaltene Lösung kann sofort zur Injektion eingesetzt werden und bleibt über den gesamten, nur durch das Abklingverhalten des $^{99m}$Tc begrenzten Zeitraum stabil.

Erfindungsgemäß wird bei der Herstellung der ersten Komponete vom Benzoylmercaptoacetyltriglycin ausgegangen, dessen Herstellung bekanntermaßen entsprechend der Vorschrift nach Fritzberg, A.R. et al.

[vgl. J. Nucl. Med. 27(1986) 111-116 ] erfolgt. Um daraus das an der Mercaptogruppe ungeschützte, für die Komponente 1 notwendige Mercaptoacetyltriglycin zu gewinnen, werden 2g Benzoyl-MAG-3 mit 30 ml wasserfreiem Methanol versetzt und unter Rühren und Einleiten von Argon werden ca. 2 mol Natriummethylat / mol Benzoyl-MAG-3 zugesetzt. Dabei geht der gesamte Feststoff in Lösung. Es wird noch ca. 30 min. bei Zimmertemperatur gerührt. Anschließend erfolgt durch Zugabe eines Kationenaustauschers in der H$^\oplus$-Form (DOWEX) die Neutralisation der Lösung. Durch sofortige Filtration durch ein Faltenfilter unter einem Schutzgas wird vom Austauscherharz abgetrennt. Das Filtrat wird lyophilisiert und das Lyophylisat durch Umkristallisation aus wäßriger Lösung gereinigt. Das so erhaltene Produkt ist bei Zimmertemperatur über 1 Jahr stabil, der Gehalt an Mercaptoverbindung beträgt >95% .

## Patentansprüche

1. Kit ( nichtradioaktive Vorstufe ) zur Herstellung einer enantiomeren Form des Nierenfunktionsdiagnostikums Technetium-99m-Mercaptoacetyltriglycin ([99mTc]-MAG-3), dadurch gekennzeichnet, daß der Kit aus zwei Komponenten besteht, deren erste ein lyophylisiertes Gemisch aus Mercaptoacetyltriglycin, einen die Oxidationsstufe +5 des Tc stabilisierenden Koliganden, ein Reduktionsmittel und ein Alkali- bzw. Erdalkalihydroxid im Molverhältnis

```
   1    :  ( 50 - 150 )  :   ( 0,1 - 0,5)   :  ( 30 - 80 )
 MAG-3  :    Koligand     : Reduktionsmittel :   Hydroxid
```

und deren zweite Komponente eine sterile Phosphatpufferlösung mit einer dem Molverhältnis MAG-3 : Hydroxid entsprechenden Menge Säure enthält.

2. Kit nach Anspruch 1, dadurch gekennzeichnet, daß als die Oxidationsstufe stabilisierender Koligand ein Salz der Weinsäure oder der Gluconsäure, als Reduktionsmittel SnCl$_2$ und als Hydroxid NaOH eingesetzt werden.

3. Kit nach Anspruch 2, dadurch gekennzeichnet, daß die erste Komponente aus einem lyophylisierten Gemisch aus
0,2 mg MAG-3
22 mg Dinatriumtartrat x 2H$_2$O
60 µg SnCl$_2$ x 2H$_2$O
1,72 mg NaOH besteht.

4. Verfahren zur Herstellung des aus zwei Komponenten bestehenden Kits nach Anspruch 1, dadurch gekennzeichnet, daß zur Herstellung der Komponente 1
Benzoyl-MAG-3 mit einem Alkoholat in alkoholischer Lösung verseift und der so gewonnene Ligand Mercaptoacetyltriglycin mit in ungeschützter Form vorliegender Mercaptogruppe mit einem Reduktionsmittel und einem die Oxidationsstufe +5 des Tc stabilisierenden Koliganden bei einem pH-Wert > 11 im angegeben Verhältnis gemischt, dann lyophylisiert und in Portionen abgefüllt wird.

5. Verfahren nach Anspruch 4, dadurch gekennzeichnet, daß das Verseifen des Benzoyl-MAG-3 mit NaOCH$_3$ in methanolischer Lösung unter einem Schutzgas erfolgt.

## Claims

1. A kit (non-radioactive precursor) for preparing an enantiomeric form of the means for kidney function diagnosis [99mTc]mercaptoacetyltriglycine ([99mTc]MAG-3) characterized thereby, that the kit consists of two components, whose first is a freeze-dried mixture consisting of:
mercaptoacetyltriglycine,
a co-ligand stabilizing the oxidation number +5 of Tc,
a reducing agent, and
an alkaline or alkaline-earth hydroxide

with a mole ratio:

1 : (50 to 150) : (0.1 to 0.5) : (30 to 80)

MAG-3 : co-ligand : reducing agent : hydroxide

and whose second is a sterile phosphate buffer solution containing an acid according to the mole ratio MAG-3 : hydroxide.

2. A kit according to patent claim 1, characterized thereby, that a salt of tartaric or gluconic acid is used as the co-ligand which stabilizes the reduction number, that $SnCl_2$ is used as reducing agent, and that NaOH is used as hydroxide.

3. A kit according to patent claim 1, characterized thereby, that the first component is a freeze-dried mixture consisting of:

0.2 mg of MAG-3

22 mg of disodium tartrate x 2 $H_2O$

66 µg of $SnCl_2$ x 2 $H_2O$ and

1.72 mg of NaOH

4. A procedure for preparing the two-component kit according to patent claim 1, characterized thereby, that for preparation of component 1 benzoyl-MAG-3 is saponified by an alcoholate in an alcoholic solution and that the obtained ligand, mercaptoacetyltriglycine with unprotected mercapto group, is mixed with a reducing agent and with a co-ligand, which stabilizes the oxidation number +5 of Tc at a pH-value of >11, in the ratio mentioned above and then freeze-dried and dispensed in fractions.

5. A procedure according to patent claim 4, characterized thereby, that the saponification of benzoyl-MAG-3 by $NaOCH_3$ in methanolic solution is carried out under an inert gas.

## Revendications

1. Kit (pré-étape non radioactive) pour la fabrication d'une forme énantiomère d'un diagnostic de fonction rénale technetium - 99m-mercaptoacétyltriglycine ($^{99m}Tc$-MAG-3), caractérisé en ce que le kit est composé de deux composants, le premier composant contenant un mélange lyophylisé de mercaptoacétyltriglycine, un coligand stabilisant le niveau d'oxydation + 5 du Tc, un agent de réduction et un hydroxide alcalin voire alcalino-terreux en relation molaire

1 : (50-150) : (0,1-0,5) : (30-80)

MAG-3 Coligand : Agent de réduction : Hydroxide

et le deuxième composant contentant une solution tampon de phosphate stérile avec une quantité d'acide correspondant à une relation molaire MAG-3 : hydroxide.

2. Kit selon la revendication 1, caractérisé en ce qu'il est utilisé un sel de l'acide tartrique ou de l'acide gluconique en tant que coligand stabilisant le niveau d'oxydation, $SnCl_2$ tant qu'agent de rédution et NaOH en tant qu'hydroxide.

3. Kit selon la revendication 1, caractérisé en ce que la premier composant est composé d'un mélange lyophylisé composé de

0,2 mg de MAG-3

22 mg de tartrate disodique x 2$H_2O$

60 µg $SnCl_2$ x 2$H_2O$

1,72 mg NaOH.

4. Procédé de fabrication du kit composé de deux composants selon la revendication 1, caractérisé en ce que pour la fabrication des composants 1 MAG-3 benzoyle est saponifié avec un alcoolat en solution alcolisée et en ce que le ligand mercaptoacétyltriglycine ainsi obtenu est mélangé avec le groupe mercaptol présent sous la forme nue avec un agent de réduction et un coligand stabilisant le niveau d'oxydation + 5 dz Tc pour une valeur pH 11 dans la relation donnée, puis lyophylisé et rempli en portions.

5. Procédé selon la revendication 4, caractérisé en ce que la saponisation du MAG 3 benzoyle s'effectue avec $HaOCH_3$ dans une solution méthanolique sous un gaz de protection.